# FASCICULE DE BREVET EUROPEEN

(11) **EP 1 268 424 B1**
(45) Date de publication et mention de la délivrance du brevet: **08.08.2007**
(21) Numéro de dépôt: 01921486.5
(22) Date de dépôt: 05.04.2001
(51) Int. Cl.: C07D 209/42, C07K 1/02

(54) **PROCEDE DE SYNTHESE DU PERINDOPRIL ET DE SES SELS PHARMACEUTIQUEMENT ACCEPTABLES**
VERFAHREN FÜR DIE SYNTHESE VON PERINDOPRIL UND SEINER PHARMAZEUTISCHEN ANNEHMBAREN SALZE
METHOD FOR SYNTHESIS OF PERINDOPRIL AND ITS PHARMACEUTICALLY ACCEPTABLE SALTS

(30) Priorité: 06.04.2000 FR 0004379
(43) Date de publication de la demande: 02.01.2003
(73) Titulaire: Les Laboratoires Servier, 92415 Courbevoie Cedex (FR)
(72) Inventeur: LANGLOIS, Pascal, F-76210 Saint Jean de la Neuville (FR); TURBE, Hugues, F-76360 Villers-Ecalles (FR)
(86) Numéro de dépôt international: PCT/FR2001/001026
(87) Numéro de publication internationale: WO 2001/058868

(56) Documents cités:
- EP-A- 0 049 658
- EP-A- 0 116 842
- EP-A- 0 308 341
- EP-A- 0 309 324
- VINCENT M ET AL: "SYNTHESIS AND ACE INHIBITORY ACTIVITY OF THE STEREOISOMERS OF PERINDOPRIL (S 9490) AND PERINDOPRILATE (S 9780)" DRUG DESIGN AND DISCOVERY,XX,HARWOOD ACADEMIC PUBLISHERS GMBH, vol. 9, no. 1, 1992, pages 11-28, XP000885876 ISSN: 1055-9612

## Description

La présente invention concerne un procédé de synthèse industrielle du perindopril de formule (I) : 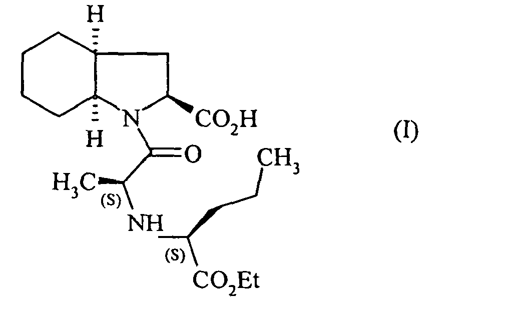 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement, et surtout avec une excellente pureté.
Le brevet EP 0 308 341 et la publication Drug Design and Discovery 1992, Vol.9, pp 11-28, décrivent tous deux la synthèse du perindopril par couplage de l'ester benzylique de l'acide (2*S*, 3a*S*, 7a*S*)-octahydroindole 2-carboxylique avec l'ester éthylique de la N-[(*S*)-l-carboxybutyl]-(*S*)-alanine, suivie de la déprotection du groupement carboxylique de l'hétérocycle par hydrogénation catalytique.
Ce procédé présente l'avantage de conduire au perindopril avec un bon rendement, à partir de matières premières dont la synthèse industrielle est décrite.
Cependant, la pureté du perindopril obtenu par ce procédé n'est pas satisfaisante, ce qui nécessite une étape de purification pour parvenir au perindopril avec une qualité permettant son emploi comme principe actif pharmaceutique.
En effet, dans les conditions décrites dans ces documents, le perindopril obtenu est contaminé dans des proportions importantes par les impuretés de formules (II) et (III) : 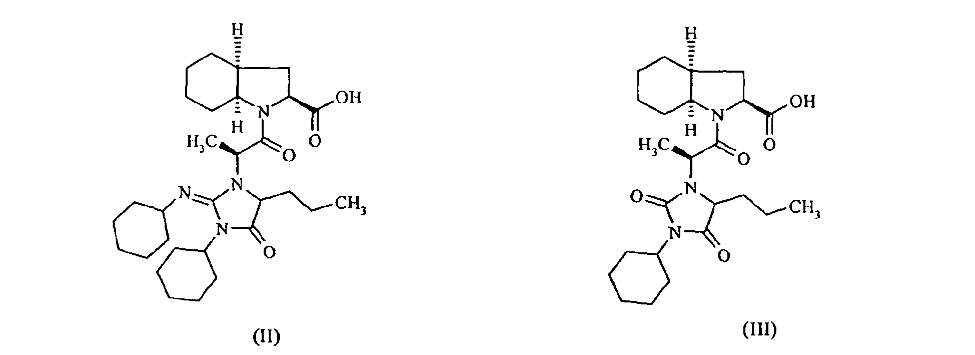 La demanderesse a présentement mis au point un nouveau procédé de synthèse industrielle conduisant au perindopril avec une pureté qui est compatible avec son utilisation comme principe actif pharmaceutique, et dont les taux en impuretés de formules (II) et (III) sont respectivement inférieurs à 0,2 % et 0,1 %.

Plus spécifiquement, la présente invention concerne un procédé de synthèse industrielle du perindopril caractérisé en ce que l'on met en réaction l'ester benzylique de formule (IV) : 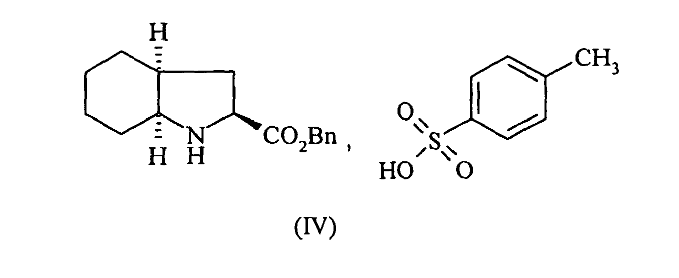 dans laquelle Bn représente le groupement benzyle,
avec le composé de formule (V) : 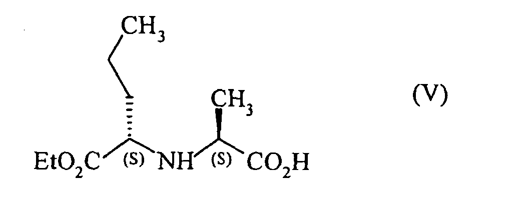 dans l'acétate d'éthyle,
en présence d'une quantité de 1-hydroxybenzotriazole comprise entre 0,4 et 0,6 mole par mole de composé de formule (IV) utilisé et d'une quantité de dicyclohexylcarbodiimide comprise entre 1 et 1,2 mole par mole de composé de formule (IV) utilisé,
en l'absence de triéthylamine ou en présence d'une quantité de triéthylamine inférieure ou égale à 0,25 mole par mole de composé de formule (IV) utilisé,
à une température comprise entre 20 et 77°C,
pour conduire après isolement au composé de formule (VI) : 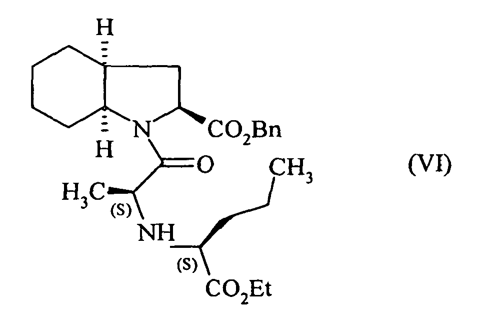 dans laquelle Bn représente le groupement benzyle, dont on déprotège le groupement carboxylique de l'hétérocycle par hydrogénation catalytique, pour conduire au perindopril de formule (I),
et que l'on transforme, si on le souhaite, en un sel pharmaceutiquement acceptable tel que le sel de tert-butylamine.

Ce procédé est particulièrement intéressant pour les raisons suivantes :
- Le couplage en milieu alcalin de l'ester benzylique de formule (IV) avec le composé de formule (V) a été décrit dans le brevet EP 0 308 341.
   Cependant, dans les conditions décrites (utilisation de 3 moles de composé de formule (V), 3 moles de triéthylamine, 3,8 moles de 1-hydroxybenzotriazole et 2,9 moles de dicyclohexylcarbodiimide par mole de composé de formule (IV) engagé), il se forme de nombreux produits secondaires.
   En particulier, le composé de formule (VI) obtenu contient dans des proportions importantes (5 à 15 %) les impuretés de formules (VII) et (VIII), qui lors de la débenzylation conduisent aux impuretés de formules (II) et (III). 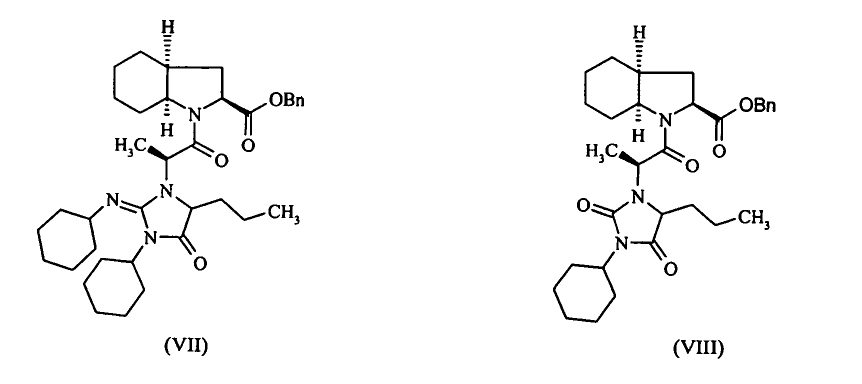
- La demanderesse a trouvé que, de manière inattendue, la diminution, voire la suppression de la triéthylamine dans l'étape de couplage permettait de limiter le taux en impuretés de formules (VII) et (VIII) dans le composé de formule (VI) à moins de 1,5 %.
- L'hydrogénation catalytique du composé de formule (VI) ainsi obtenu conduit au perindopril avec une pureté bien meilleure, et notamment avec des taux en impuretés de formules (II) et (III) inférieurs respectivement à 0,2 % et 0,1 %.
- De plus, la diminution, dans l'étape de couplage, de la quantité en composé de formule (V), en 1-hydroxybenzotriazole et en dicyclohexylcarbodiimide, permettent d'obtenir un rendement aussi bon en composé de formule (VI) qu'avec des quantités de réactifs plus importantes, rendant ainsi le procédé beaucoup plus avantageux à l'échelle industrielle.

Les exemples ci-dessous illustrent l'invention.

### Exemple 1: (2S, 3aS, 7aS)-1-{(2S)-2[(1S)-1-(Ethoxycarbonyl)-butylamino]-propionyl}-octahydro-1H-indole-2-carboxylate de benzyle :

Dans un réacteur sous agitation sont introduits 1 kg de paratoluènesulfonate de l'ester benzylique de l'acide (2*S*, 3a*S*, 7a*S*)-octahydroindole 2-carboxylique, 0,06 kg de triéthylamine, 4,6 1 d'acétate d'éthyle puis, après 10 mn d'agitation à température ambiante, 0,52 kg de N-[(*S*)-carbéthoxy-1-butyl]-(*S*)-alanine, 0,15 kg de 1-hydroxybenzotriazole et 0,5 kg de dicylohexylcarbodiimide. Le mélange hétérogène est ensuite porté à 30°C pendant 3h sous bonne agitation, puis il est refroidi à 0°C et filtré.
Le filtrat est ensuite lavé, puis évaporé à sec pour conduire au produit attendu avec un rendement de 92%.

### Exemple 2 : Acide (2S, 3aS, 7aS)-1-{(2S)-2-[(1S)-1-(éthoxycarbonyl)-butylamino]-propionyl}-octahydro-1H-indole-2-carboxylique :

Le résidu obtenu dans le stade précédent (1 kg) est mis en solution dans 1 1 de méthylcyclohexane et transféré dans un hydrogénateur, puis 0,13 kg de charbon palladié à 5% en suspension dans 0,41 de méthylcyclohexane sont ajoutés, suivis de 3,2 1 d'eau.
Le mélange est ensuite hydrogéné sous une pression de 0,5 bar, à une température comprise entre 15 et 30°C, jusqu'à absorption de la quantité théorique d'hydrogène.

Après filtration du catalyseur, la phase aqueuse du filtrat est lavée par du méthylcyclohexane, puis lyophilisée pour conduire au produit attendu avec un rendement de 94%.

### Exemple 3 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique :

Le lyophilisat obtenu dans le stade précédent (1 kg) est mis en solution dans 14 1 d'acétate d'éthyle, puis 0,2 kg de tert-butylamine et 2 1 d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95%.

## Revendications

1. Procédé de synthèse industrielle du perindopril de formule (I) 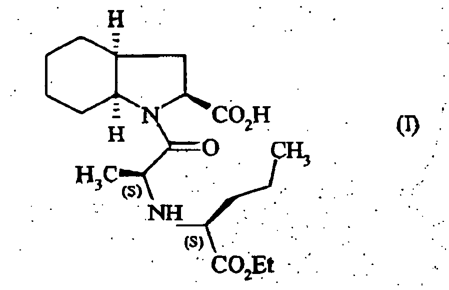 et de ses sels pharmaceutiquement acceptables, **caractérisé en ce que** l'on met en réaction l'ester benzylique de formule (IV): 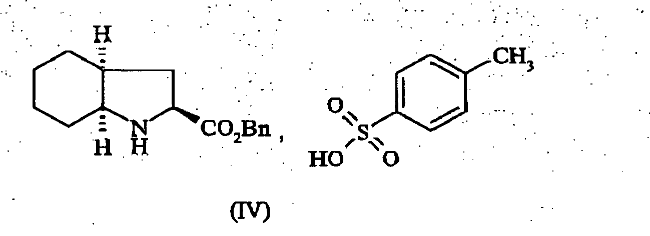 dans laquelle Bn représente le groupement benzyle,
avec lé composé de formule (V): 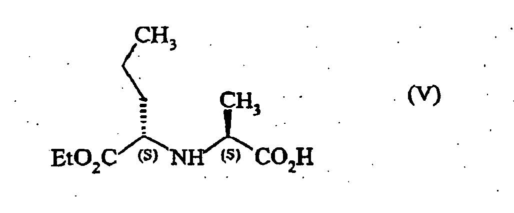 dans l'acétate d'éthyle,
en présence d'une quantité de 1-hydroxybenzotriazole comprise entre 0,4 et 0,6 mole par mole de composé de formule (IV) utilisé et d'une quantité de dicyclohexylcarbodiimide comprise entre 1 et 1,2 mole par mole de composé de formule (IV) utilisé,
en l'absence de triéthylamine ou en présence d'une quantité de triéthylamine inférieure ou égale à 0,25 mole par mole de composé de formule (IV) utilisé,
à une température comprise entre 20 et 77°C, pour conduire après isolement au composé de formule (VI) : 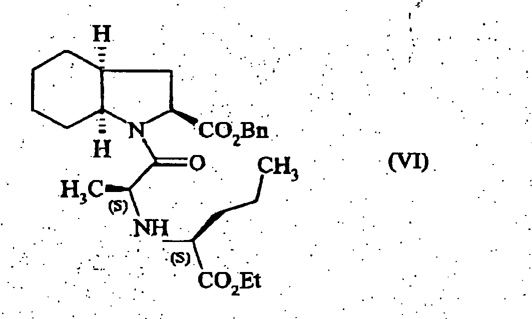 dans laquelle Bn représente le groupement benzyle,
dont on déprotège le groupement carboxylique de l'hétérocycle par hydrogénation catalytique, pour conduire au perindopril de formule (I),
que l'on transforme, si on le souhaite, en un sel pharmaceutiquement acceptable.

2. Procédé de synthèse selon la revendication 1 du perindopril sous sa forme de sel de tert-butylamine.

3. Procédé de synthèse selon l'une quelconque des revendications 1 ou 2, **caractérisé en ce que** le composé de formule (VI) est obtenu avec un taux en impuretés de formules (VII) et (VIII) qui est inférieur à 1,5%.

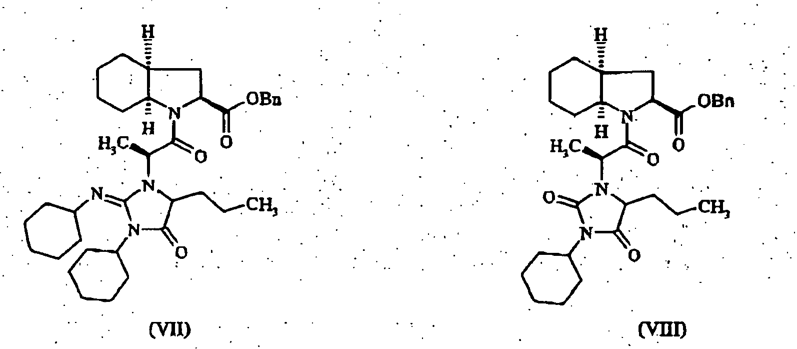

4. Procédé de synthèse selon l'une quelconque des revendications 1 à 3, **caractérisé en ce que** le perindopril est obtenu avec des taux en impuretés de formules (II) et (III) qui sont respectivement inférieurs à 0,2 % et 0,1%.

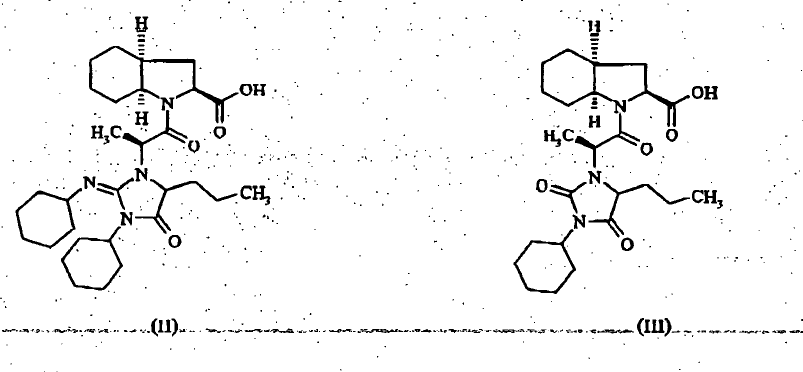

## Claims

1. Process for the industrial synthesis of perindopril of formula (I) 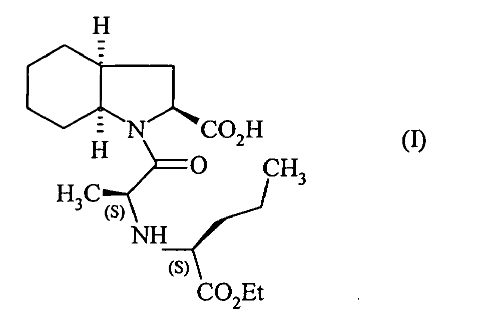 and pharmaceutically acceptable salts thereof, **characterised in that** the benzyl ester of formula (IV) : 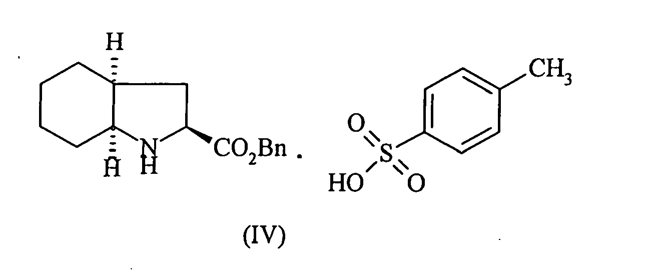 wherein Bn represents the benzyl group,
is reacted with the compound of formula (V) : 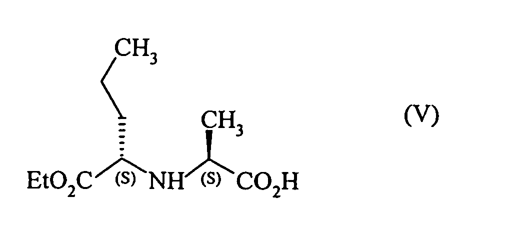 in ethyl acetate,
in the presence of an amount of from 0.4 to 0.6 mol of 1-hydroxybenzotriazole per mol of compound of formula (IV) employed and in the presence of an amount of from 1 to 1.2 mol of dicyclohexylcarbodiimide per mol of compound of formula (IV) employed, in the absence of triethylamine or in the presence of an amount of triethylamine that is less than or equal to 0.25 mol per mol of compound of formula (IV) employed,
at a temperature of from 20 to 77°C,
to yield, after isolation, the compound of formula (VI) : 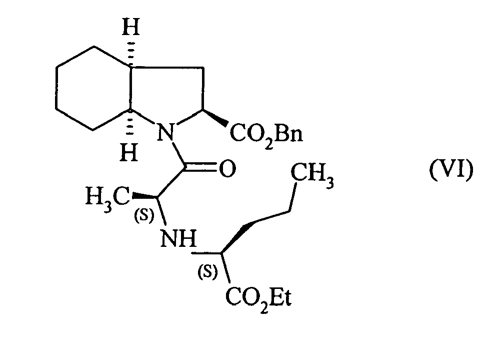 wherein Bn represents the benzyl group,
the carboxylic group of the heterocycle of which is deprotected by catalytic hydrogenation to yield perindopril of formula (I),
which is converted, if desired, into a pharmaceutically acceptable salt.

2. Process according to claim 1 for the synthesis of perindopril in the form of its tert-butylamine salt.

3. Synthesising process according to claim 1 or 2, **characterised in that** the compound of formula (VI) is obtained with a level of impurities of formulae (VII) and (VIII) that is less than 1.5 %

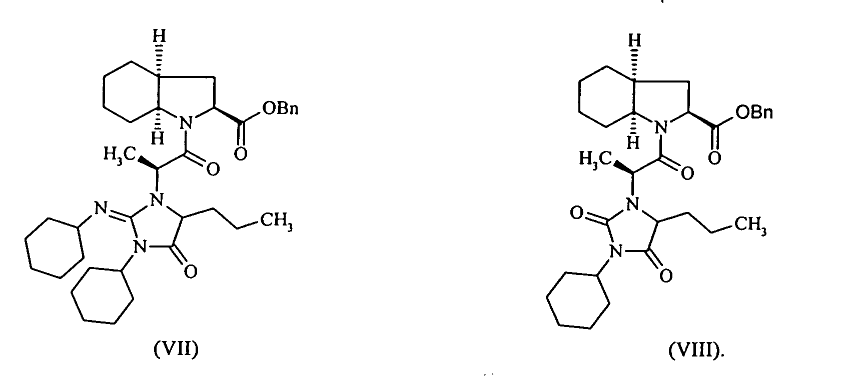

4. Synthesising process according to any one of claims 1 to 3, **characterised in that** the perindopril is obtained with levels of impurities of formulae (II) and (III) that are less than 0.2 % and 0.1 %, respectively

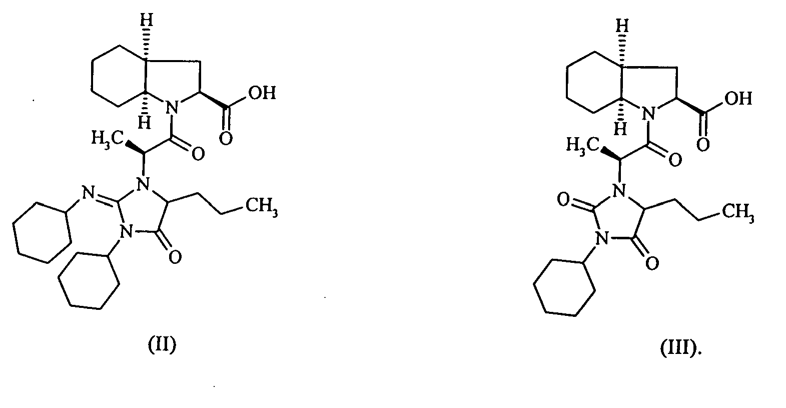

## Patentansprüche

1. Verfahren zur technischen Synthese von Perindopril der Formel (1): 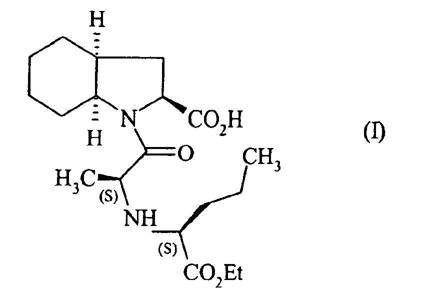 und von seinen pharmazeutisch annehmbaren Salzen, **dadurch gekennzeichnet, dass** man den Benzylester der Formel (IV): 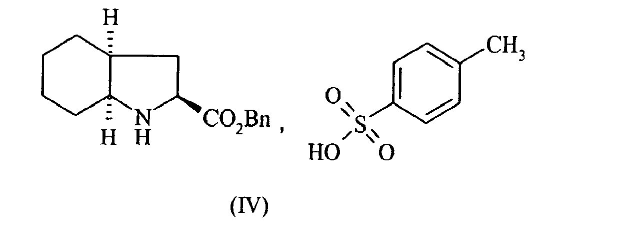 in der Bn die Benzylgruppe bedeutet,
mit der Verbindung der Formel (V): 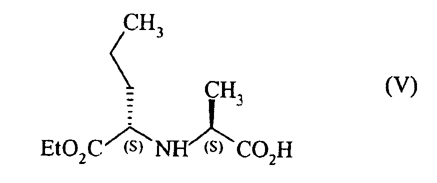 in Ethylacetat umsetzt,
in Gegenwart einer Menge von 1-Hydroxybenzotriazol zwischen 0,4 und 0,6 Mol pro Mol der verwendeten Verbindung der Formel (IV) und einer Menge von Dicyclohexylcarbodiimid zwischen 1 und 1,2 Mol pro Mol der verwendeten Verbindung der Formel (V),
in Abwesenheit von Triethylamin oder in Gegenwart einer Menge von Triethylamin, die gleich oder geringer ist als 0,25 Mol pro Mol der verwendeten Verbindung der Formel (IV),
bei einer Temperatur zwischen 20 und 77 °C.
so dass man nach der Isolierung die Verbindung der Formel (V1): 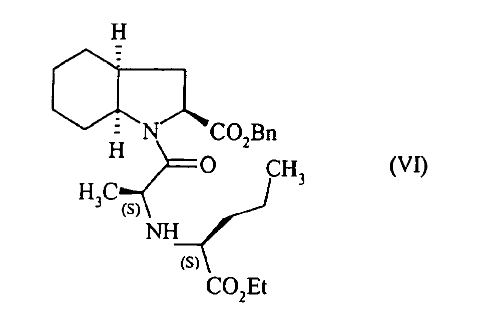 erhält, in der Bn die Benzylgruppe bedeutet,
von der man die Schutzgruppe der Carboxylgruppe des Heterocyclus durch katalytische Hydrierung entfernt unter Bildung von Perindopril der Formel (I), welche man gewünschtenfalls in ein pharmazeutisch annehmbares Salz überführt.

2. Verfahren zur Synthese von Perindopril nach Anspruch 1 in Form seines tert.-Butvlaminsalzes.

3. Syntheseverfahren nach einem der Ansprüche 1 oder 2, **dadurch gekennzeichnet, dass** man die Verbindung der Formel (VI) mit einem Gehalt an Verunreinigungen der Formeln (VII) und (VIII) von weniger als 1,5 % herstellt:

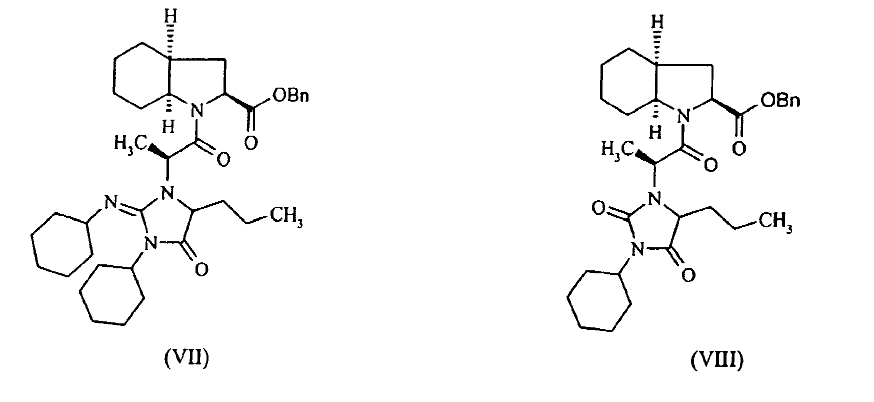

4. Syntheseverfahren nach einem der Ansprüche 1 bis 3, **dadurch gekennzeichnet, dass** man Perindopril mit einem Gehalt an Verunreinigungen der Formeln (II) und (III), die geringer sind als 0.2 % bzw. 0,1 % erhält:
